# EUROPEAN PATENT SPECIFICATION

(11) **EP 2 819 719 B1**
(45) Date of publication and mention of the grant of the patent: **06.01.2016**
(21) Application number: 13720034.1
(22) Date of filing: 26.02.2013
(51) Int. Cl.: A61M 1/16, A61M 1/36

(54) **APPLIANCE CONNECTABLE TO A DEVICE FOR THE EXTRACORPOREAL OXYGENATION OF BLOOD**
AN EINE VORRICHTUNG ANSCHLIESSBARE EINRICHTUNG ZUR EXTRAKORPORALEN OXYGENIERUNG VON BLUT
APPAREIL CONNECTABLE À UN DISPOSITIF POUR L'OXYGÉNATION EXTRA-CORPORELLE DU SANG

(30) Priority: 01.03.2012 IT MO20120052
(43) Date of publication of application: 07.01.2015
(73) Proprietor: Eurosets S.r.l., 41036 Medolla (MO) (IT)
(72) Inventor: GHELLI, Nicola, 40018 S. Pietro in Casale (BO) (IT); PETRALIA, Antonio, 48022 Lugo (RA) (IT)
(74) Representative: Brunacci, Marco
(86) International application number: PCT/IB2013/051546
(87) International publication number: WO 2013/128375

(56) References cited:
- WO-A2-01/26709
- US-A1- 2003 215 356
- US-B1- 6 368 557

## Description

### Technical Field

The present invention relates to an appliance connectable to a device for the extracorporeal oxygenation of a patient's blood.

### Background Art

As is known, in extracorporeal blood circulation practised by bypassing the heart and lungs of the patient during cardiac arrest and open-heart surgical operations, or to temporarily aid the patient's circulation and breathing in clinical conditions of acute heart failure and/or respiratory failure (ECMO), blood oxygenation devices are used which are suitable for enriching the blood with oxygen and, from a functional viewpoint, substituting the patient's lungs. The blood oxygenation devices of known type, commonly called "oxygenators", are composed of a containment body with a venous blood inlet connection and an arterial blood outlet connection, inside which a work gas is conveyed suitable for yielding oxygen to the blood and receiving carbon dioxide from this.

A particular type of known oxygenator is the "hollow fibre" type, meaning it is distinguished by a bundle of hollow fibres arranged inside the body of the oxygenator and made up of material semi-permeable to the gases but not to the liquids. These hollow fibres are therefore internally crossed by the work gas and are skimmed on the outside by the blood arriving from the patient.

During operation therefore, the blood which crosses the containment body skims the hollow fibres and exchanges oxygen and carbon dioxide with these. More in detail, the blood that crosses the oxygenator takes oxygen from the gas contained in the hollow fibres and yields carbon dioxide to this.

The known oxygenators therefore also comprise an inlet channel and an outlet channel of the work gas, where the gas that enters is made up of oxygen, possibly mixed with air, and that which exits is made up of oxygen and carbon dioxide.

Generally, in the known oxygenation devices, the pressure of the blood, which is around 200 mmHg, is higher than that of the gas, which is around 5-15.

These known oxygenators have a number of drawbacks.

These in fact, in view of the amount of oxygen they are able to yield to the blood in the unit of time, require large exchange surfaces in order to achieve the correct oxygenation of the blood.

This, on the one hand affects the dimensions and overall dimensions of the known oxygenators and on the other involves a risk of traumas for the blood, which can cause chemical-physical alterations of the blood as it flows through the oxygenator which increase with the increase in size of the work gas exchange surface.

Patent document WO 01/26709 discloses an apparatus for blood oxygenation.

### Description of the Invention

The main aim of the present invention is to provide a device connectable to a blood oxygenator which allows to obtain an oxygenation which is considerably more efficient than known devices.

Within this aim, one object of the present invention is to reduce the surface of blood exchange with the work gas compared to known devices, in order to curb overall dimensions and reduce the risk of traumas for the treated blood. In particular, in the hollow-fibre oxygenators, the present invention intends reducing the quantity of hollow fibres used.

Another object of the present invention is to provide a device connectable to a blood oxygenator which allows to overcome the mentioned drawbacks of the state of the art in the ambit of a simple, rational, easy and effective to use as well as low cost solution.

The above objects are achieved by the present appliance connectable to a device for the extracorporeal oxygenation of blood of the type comprising at least an inlet connection and an outlet connection for the blood, at least an inlet channel and an outlet channel for a work gas intended to supply oxygen to blood and/or remove CO₂ from the same, characterized by the fact that it comprises:
- first sensor means which can be positioned along at least one between the inlet and outlet connections of blood to read at least a characteristic blood value corresponding to the blood pressure along one between said inlet connection and said outlet connection or to the blood pressure interval between them;
- second sensor means which can be positioned along at least one between the inlet and outlet channels of gas to read at least a characteristic gas value corresponding to the gas pressure along one between said inlet channel and said outlet channel or to the gas pressure interval between them;
- variation means for varying at least the gas pressure along the inlet channel and/or along the outlet channel, wherein said variation means are operatively connected to said first and second sensor means and are programmed to make the difference between said characteristic gas value read by said second sensor means and said characteristic blood value read by said first sensor means substantially equal to at least a predefined value.

### Brief Description of the Drawings

Other characteristics and advantages of the present invention will become more evident from the description of a preferred, but not sole, embodiment of an appliance connectable to a device for the extracorporeal oxygenation of blood, illustrated purely as an example but not limited to the annexed drawings in which:

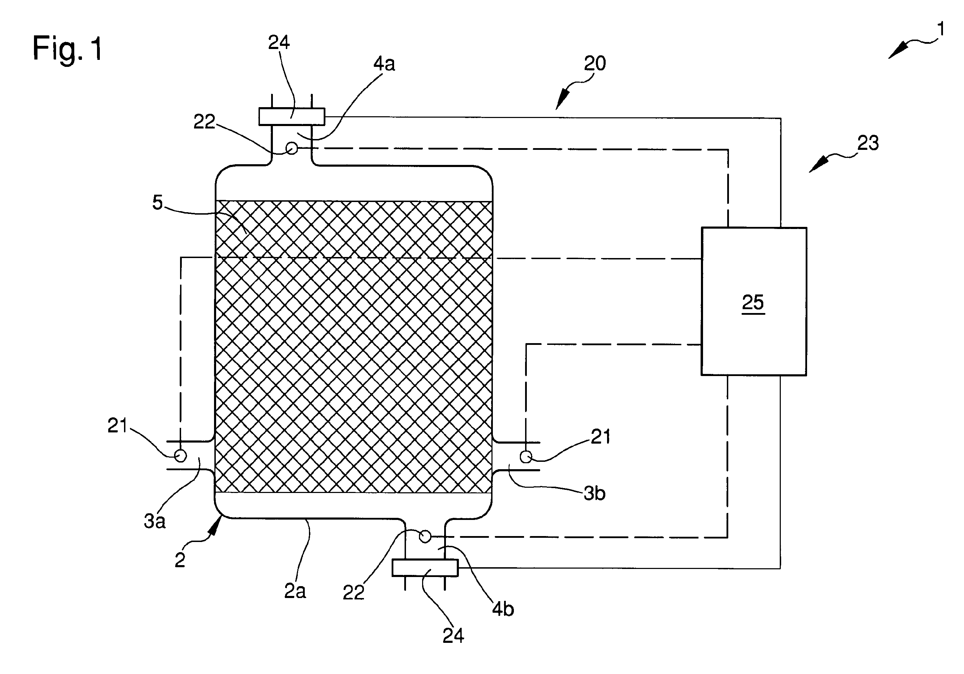

Figure 1 is a schematic view of an apparatus for the extracorporeal oxygenation of blood.

### Embodiments of the Invention

With particular reference to such figure, globally indicated by 1 is an apparatus for blood oxygenation.

The apparatus 1 comprises a device 2 for the extracorporeal oxygenation of blood and an appliance 20 according to the invention, which is connected to the device 2 itself.

The device 2 is of the type known to the technician in the sector and is suitable for infusing oxygen in the blood arriving from the patient before its reinfusion in the patient him/herself. As is known, the device 2 comprises a casing 2a which defines an oxygenation chamber inside which the oxygenation occurs of the blood arriving from the patient.

The casing 2a has an inlet connection 3a, which receives the blood to be oxygenated arriving from the patient (called venous blood), and an outlet connection 3b, through which the oxygenated blood comes out (called arterial blood) intended to be re-infused in the patient.

The inlet connection 3a can be connected to other devices of the apparatus 1, not shown in the illustrations, such as a supply pump suitable for maintaining blood circulation, e.g., a roller pump, and a thermal conditioning device suitable for keeping blood temperature under control by adding or subtracting heat, e.g., a heat exchanger.

The outlet connection 3b can be connected to a filtering device, this too not shown in figure 1, suitable for removing any bubbles of air or of other gases, before the treated blood is reintroduced into the patient's body.

The casing 2a therefore has an inlet channel 4a and an outlet channel 4b for the work gas. More in detail, the inlet channel 4a is crossed by oxygen, possibly mixed with air, and the outlet channel 4b is crossed by oxygen and carbon dioxide.

Inside the device 2, and more in particular, inside the oxygenation chamber, are arranged flow means 5 for the flow of a work gas, generally oxygen or oxygen mixed with air, suitable for yielding oxygen to the blood and removing the CO2 from the blood.

Preferably, the flow means 5 are composed of hollow-fibre bundles semipermeable to the gas, which are internally crossed by oxygen and externally skimmed by the blood. The extremities of these hollow-fibre bundles are drowned in polyurethane-resin rings, called "potting"., and are open towards the inlet channel 4a and the outlet channel 4b respectively. During the operation of the oxygenator 2, the blood therefore exchanges oxygen and carbon dioxide with the gas circulating inside the hollow fibres. Alternative embodiments cannot however be ruled out that contemplate the use of a different type of flow means 5 known to the technician in the sector.

The device 2 therefore presents two compartments separated from one another by the flow means 5, i.e., the gas compartment, comprising the channels 4a and 4b, and the blood compartment, comprising the connections 3a and 3b. According to the invention, the appliance 20 comprises at least first sensor means 21 which can be positioned along at least one between the inlet connection 3a and the outlet connection 3b to read a characteristic blood value Vb.

The characteristic blood value Vb, hereinafter for brevity blood value Vb, corresponds to the blood pressure along one between the inlet connection 3 a (hereinafter blood inlet pressure pbin) and the outlet connection 3b (hereinafter blood outlet pressure pbout) or to the blood pressure interval Δpb between the inlet connection and the outlet connection themselves.

The first sensor means 21 can therefore be arranged along just one of the two connections 3a, 3b, in which case the blood value Vb corresponds to the inlet pressure or the outlet pressure of the blood pbin, pbout, or along both the connections 3a, 3b, in which case the blood value Vb corresponds to the pressure interval Δpb between these.

Preferably, the first sensor means 21 are made up of pressure sensors, though the use of different sensors known to the technician in the sector cannot be ruled out.

The appliance 20 also comprises second sensor means 22 which can be arranged along at least one between the inlet channel 4a and the outlet channel 4b to read a characteristic gas value Vg.

The characteristic gas value Vg, hereinafter for brevity gas value Vg, corresponds to the gas pressure along one between the inlet channel 4a (hereinafter gas inlet pressure pgin) and the outlet channel 4b (hereinafter gas outlet pressure pgout) or to the gas pressure interval Δpg between the inlet channel and the outlet channel themselves.

The second sensor means 22 can therefore be arranged along just one of the two channels 4a, 4b, in which case the gas value Vg corresponds to the inlet pressure or the outlet pressure of the gas pgin, pgout, or along both the channels 4a, 4b, in which case the gas value Vg corresponds to the gas pressure interval Δpg between these.

In the case of a device 2 of the hollow-fibre type, the second sensor means 22 arranged along the inlet channel 4a and/or along the outlet channel 4b are positioned upstream and downstream of the resin rings respectively, in which the extremities of the hollow-fibres themselves are drowned.

Suitably, the second sensor means 22 are of the same type as the first sensor means 21.

The appliance 20 also comprises variation means 23 suitable for modifying at least the gas pressure along at least one between the inlet channel 4a and the outlet channel 4b.

According to the invention, the variation means 23 are operatively connected to the first and second sensor means 21 and 22 and are programmed to make the difference between the gas value Vg and the blood value Vb substantially equal to at least a predefined value.

More in particular, the variation means 23 are programmed to make the difference between the gas value Vg and the blood value Vb substantially equal to a single predefined value or to a plurality of predefined values, the latter in practice defining an interval of predefined values and the variation means 23 being suitable for maintaining the difference between the gas value Vg and the blood value Vb within such interval.

By the term "substantially" used here is meant less the tolerance error of the instruments used to measure blood pressure.

Advantageously, the first sensor means 21 and the second sensor means 22 are arranged along the outlet connection 3b of the blood and along the inlet channel 4a of the gas respectively and, consequently, in this embodiment, the blood value Vb and the gas value Vg correspond to the blood outlet pressure pbout and to the gas inlet pressure pgin respectively.

In an alternative embodiment, the first sensor means 21 are arranged along both the connections 3a, 3b and the second sensor means 22 are arranged along both the channels 4a, 4b and, consequently, the blood value Vb and the gas value Vg correspond to the blood pressure interval Δpb (i.e., the difference between the pressure read in correspondence to the inlet connection 3a and that read in correspondence to the outlet connection 3b) and to the gas pressure interval Δpg (i.e., the difference between the pressure read in correspondence to the inlet channel 4a and that read in correspondence to the outlet channel 4b) respectively.

With special reference to the case wherein the first and the second sensor means 21 and 22 are suitable for reading the blood outlet pressure pbout and the gas inlet pressure pgin, the aforementioned predefined value is higher or the same as -140 mmHg and preferably higher or the same as 80mmHg. In the particular case of the flow means 5 being composed of hollow fibres of the type known at the time of the filing, the predefined value is advantageously between 80 mmHg and 130 mmHg.

The upper limit of the reference value is defined by the intrinsic properties of the materials used and substantially corresponds to the value at which the blood gurgling phenomenon occurs. Maximum values cannot therefore be ruled out which are considerably higher than those mentioned in the event of materials other than hollow fibres being used.

Laboratory tests have shown that, with a difference between the gas inlet pressure pgin and the blood outlet pressure pbout of -114mmHg, the quantity of oxygen yielded to the blood equals 272 ml/min, while with a difference of 80 mmHg, the quantity of oxygen yielded to the-blood is equal to 453 mmHg. It is therefore evident how, blood pressure being equal, an increase in gas pressure results in a considerable increase in the quantity of oxygen yielded to the blood in the unit of time.

As mentioned above, by keeping the pressure difference between the gas compartment and the blood compartment below a maximum predefined value and depending on the material used to convey the gas itself, the gurgling phenomenon, i.e., the formation of air bubbles in the blood, is avoided. Advantageously, the variation means 23 comprise at least regulation means 24 of the gas flow section, positionable along the inlet channel 4a and/or along the outlet channel 4b. The regulation means 24 are, e.g., made up of a valve or the like, suitable for modifying the flow section of the relative channel, thus causing an alteration in the pressure along same. Preferably, the regulation means 24 are positioned along the outlet channel 4b, so as to allow an increase in pressure in the area upstream of the regulation means themselves. Alternative embodiments cannot however be ruled out which provide for the presence of regulation means 24 both along the outlet channel 4b and along the inlet channel 4a.

Preferably, the second sensor means 22 are arranged along the inlet channel 4a and the outlet channel 4b downstream and upstream respectively of the regulation means 24 positioned along the same channels 4a arid 4b, so as to determine the effect caused by the regulation means themselves on the pressures of the gas acting directly on the blood made to circulate inside the device 2. Alternatively, or in addition, the variation means 23 can also comprise gas flow regulation means (not shown in the illustrations), which are suitable for causing an increase or a decrease in gas pressure due to an increase or a decrease respectively in the flow of same, the flow section remaining substantially unchanged. These flow regulation means permit not only regulating the gas pressure, but also preventing a rise in the quantity of CO2 removed. Advantageously, the appliance 20 also comprises command and control means 25 of the variation means 23.

More in particular, the command and control means 25 comprise an electronic unit with a microprocessor and a connecting circuit of the electronic unit to the variation means 23.

The command and control means 25 have therefore a first and a second inlets operatively connected to the first and second sensor means 21 and 22 respectively, and at least an outlet operatively connected to the variation means 23.

The command and control means 25 are designed to identify the blood and gas values Vb and Vg depending on the data received from the first and second sensor means 21 and 22, to calculate their difference, to compare such difference with the predefined value/s and to send an indicative signal of the difference thus calculated to the variation means 23.

Suitably, the command and control means 25 also comprise at least a programmable memory wherein the predefined values can be entered.

More in detail, if the difference calculated between the read gas value Vg and the blood value Vb is higher than the maximum predefined value, the command and control means 25 intervene on the variation means 23, sending these a relative signal, in such a way that these reduce the gas value Vg, i.e. to reduce the read gas pressure or the gas pressure interval Δpg at the extremities of the device 2, e.g., by increasing the gas flow section by means of the regulation means 24.

In the same way, in the event of the difference calculated between the read gas value Vg and the blood value Vb being lower than the predefined minimum value, the command and control means 25 intervene on the variation means 23 in such a way that these increase the gas value Vg, i.e. to increase the read gas pressure or the gas pressure interval Δpg at the extremities of the device 2, e.g., by narrowing the gas flow section by means of the regulation means 24.

Suitably, the second sensor means 22 are connected in feedback to the command and control means 25, in order to make sure the difference between the gas value Vg and the blood value Vb continues to be equal to at least one of the predefined values and, if necessary, intervene on the gas flow section along the inlet channel 4a and/or outlet channel 4b by means of the regulation means 24.

The first and the second sensor means 21 and 22 allow taking measurements at regular time intervals or in continuous mode. This way, the gas value Vg can be adapted at any time to the blood value Vb read from time to time.

In a particular embodiment of the appliance 20, the variation means 23 also comprise at least one active element positionable in correspondence to the supply pump to change the flow of blood entering the device 2. Suitably, this active element is also operatively connected to the first and the second sensor means 21 and 22. More in detail, in this embodiment, the command and control means 25 comprise a second outlet operatively connected to the active element positioned on the supply pump. The command and control means 25 are suitable for operating the active element in particular in the case of the difference between the gas value Vg and the blood value Vb exceeding the predefined maximum value, so as to return such difference, as quickly as possible, below such value and thus prevent blood gurgling.

Preferably, the appliance 20 also comprises gas heating means, not shown in the illustrations, suitable for increasing the working temperature of the gas introduced into the device 2 to increase the diffusion of same in the blood.

The present disclosure also relates to a method for the oxygenation of blood, comprising the following phases of:
- providing an oxygenation device 2 having at least an inlet connection 3a of the blood to be oxygenated arriving from a patient, an outlet connection 3b of the oxygenated blood intended to be reintroduced into the patient, at least an inlet channel 4a and an outlet channel 4b for a work gas intended to yield oxygen to blood before its reintroduction into the patient and having flow means 5 for the gas placed between said inlet channel 4a and said outlet channel 4b;
- taking blood from a patient and conveying it into the oxygenation device 2;
- oxygenating the blood taken from the patient by means of the oxygenation device 2;
- reintroducing the oxygenated blood into the patient;
- reading a characteristic blood value Vb corresponding to the blood pressure pbin, pbout along one between the inlet connection 3a and the outlet connection 3b or to the blood pressure interval Δpb between them;
- reading a characteristic gas value Vg corresponding to the gas pressure pgin, pgout along one between the inlet channel 4a and the outlet channel 4b or to the gas pressure interval Δpg between them;
- calculating the difference between the read characteristic gas value Vg and the characteristic blood value Vb;
- imposing that the difference calculated between the read characteristic gas and blood values Vg, Vb is substantially equal to at least a predefined value.

More in particular, the above-mentioned imposing phase comprises at least one phase of gas pressure variation in correspondence to at least one between the inlet channel 4a and the outlet channel 4b, e.g., by varying the relative gas flow sections or the flow rate of same.

More in detail, the imposing phase contemplates the difference between the read characteristic gas value Vg and the characteristic blood value Vb being between a predefined minimum value and a maximum value.

It has in fact been ascertained how the described invention achieves the proposed objects and in particular the fact is underlined that by constantly controlling the difference between the gas and blood pressure intervals inside the oxygenator, it is possible to obtain a hyper-oxygenation of the blood and at the same time avoid the gurgling of same.

The present invention therefore allows considerably reducing the overall dimensions of the oxygenator with respect to the known devices, the degree of blood oxygenation being equal.

It therefore follows that the device according to the invention permits reducing, compared to known oxygenators, the exchange surface between blood and gas, thus limiting the risk of traumas to the treated blood.

## Claims

1. Appliance (20) connectable to a device (2) for the extracorporeal oxygenation of blood of the type comprising at least an inlet connection (3a) and an outlet connection (3b) for the blood, at least an inlet channel (4a) and an outlet channel (4b) for a work gas intended to supply oxygen to blood and/or remove CO₂ from the same, **characterized by** the fact that it comprises:
- first sensor means (21) which can be positioned along at least one between the inlet and outlet connections (3a, 3b) of blood to read at least a characteristic blood value (V_{b}) corresponding to the blood pressure (p_{bin}, p_{bout}) along one between said inlet connection (3a) and said outlet connection (3b) or to the blood pressure interval (Δp_{b}) between them;
- second sensor means (22) which can be positioned along at least one between the inlet and outlet channels (4a, 4b) of gas to read at least a characteristic gas value (V_{g}) corresponding to the gas pressure (p_{gin}, p_{gout}) along one between said inlet channel (4a) and said outlet channel (4b) or to the gas pressure interval (Δp_{g}) between them;
- variation means (23) for varying at least the gas pressure along the inlet channel (4a) and/or along the outlet channel (4b), wherein said variation means (23) are operatively connected to said first and second sensor means (21, 22) and are programmed to make the difference between said characteristic gas value (Vg) read by said second sensor means (22) and said characteristic blood value (V_{b}) read by said first sensor means (21) substantially equal to at least a predefined value.

2. Appliance (20) according to claim 1, **characterized by** the fact that said first sensor means (21) can be positioned at least along said blood outlet connection (3b) and by the fact that said second sensor means (22) can be positioned at least along said gas inlet channel (4a), said characteristic gas value (V_{g}) and characteristic blood value (V_{b}) corresponding to the gas inlet pressure (p_{gin}) along said inlet channel (4a) and to the blood outlet pressure (p_{bout}) along said outlet connection (3b), respectively.

3. Appliance (20) according to claim 1 or 2, **characterized by** the fact that said first sensor means (21) can be positioned along the blood inlet and outlet connections (3a, 3b) to read the blood pressure interval (Δp_{b}) between them, that said second sensor means (22) can be positioned along the gas inlet and outlet channels (4a, 4b) to read the gas pressure interval (Δp_{g}) between them, said characteristic gas value (Vg) and characteristic blood value (V_{b}) corresponding to said gas pressure interval (Δp_{g}) and to said blood pressure interval (Ap_{b}), respectively.

4. Appliance (20) according to one or more of the preceding claims, **characterized by** the fact that said predefined value is substantially higher than or equal to 80 mmHg.

5. Appliance (20) according to one or more of the preceding claims, **characterized by** the fact that it comprises command and control means (25) of said variation means (23).

6. Appliance (20) according to claim 5, **characterized by** the fact that said command and control means (25) have at least a first and a second inlet operatively connected to said first and second sensor means (21, 22), respectively, and at least an outlet operatively connected to said variation means (23), said command and control means (25) being intended to calculate the difference between said characteristic gas value (V_{g}) and said characteristic blood value (V_{b}) read by said first and second sensor means (21, 22) respectively, and to send an indicative signal of said difference to said variation means (23).

7. Appliance (20) according to one or more of the preceding claims, **characterized by** the fact that said second sensor means (22) are connected in feedback to said command and control means (25).

8. Appliance (20) according to one or more of the preceding claims, **characterized by** the fact that said variation means (23) comprise regulation means (24) for regulating the flow section of at least one between said inlet channel (4a) and said outlet channel (4b).

9. Appliance (20) according to claim 8, **characterized by** the fact that said second sensor means (22) can be positioned along said gas inlet and/or outlet channel (4a, 4b), downstream and upstream respectively of the regulation means (24) with respect to the work gas flow direction.

10. Appliance (20) according to one or more of the preceding claims, **characterized by** the fact that said variation means (23) comprise regulation means for regulating the gas flow through said inlet and outlet channels (4a, 4b).

11. Appliance (20) according to one or more of the preceding claims, **characterized by** the fact that said variation means (23) comprise at least one active element which can be positioned on a supply pump supplying blood to the oxygenation device (2) and suitable for modifying the blood flow through said inlet and outlet connections (3a, 3b).

12. Appliance (20) according to one or more of the preceding claims, **characterized by** the fact that it comprises heating means for heating the gas entering into said oxygenation device (2).

13. Apparatus comprising :
- a device (2) for blood oxygenation having at least an inlet connection (3a) and an outlet connection (3b) for the blood, at least an inlet channel (4a) and an outlet channel (4b) of a work gas intended to supply oxygen to the blood and/or to remove CO₂ from the same, between said inlet channel (4a) and said outlet channel (4b) being arranged flow means (5) for the gas;
- at least a supply pump suitable for moving the blood through said oxygenation device (2);
said apparatus also comprising an appliance (20) according to one or more of the claims from 1 to 12, said appliance (20) being connected to said oxygenation device (2) downstream of said supply pump.

## Patentansprüche

1. Gerät (20), das angeschlossen werden kann an eine Einrichtung (2) für die extrakorporale Sauerstoffanreicherung von Blut, in der Bauart mit wenigstens einem Einlassanschluss (3a) und einem Auslassanschluss (3b) für das Blut, wenigstens einem Einlasskanal (4a) und einem Auslasskanal (4b) für ein Arbeitsgas, welches Sauerstoff dem Blut zuführen und/oder CO₂ aus demselben abführen soll, **gekennzeichnet durch** die Tatsache, dass dieses umfasst:
- eine erste Sensoreinrichtung (21), welche an wenigstens dem Einlass- oder dem Auslassanschluss (3a, 3b) für Blut positioniert werden kann, um wenigstens einen charakteristischen Blutwert (V_{b}) zu lesen, der mit dem Blutdruck (p_{bin},p_{bout}) in dem Einlassanschluss (3a) und dem Auslassanschluss (3b) oder mit der Blutdruckdifferenz (Δp_{b}) zwischen diesen korrespondiert;
- eine zweite Sensoreinrichtung (22), welche entlang wenigstens dem Einlass- oder Auslasskanal (4a, 4b) für Gas positioniert werden kann, um wenigstens einen charakteristischen Gaswert (Vg) zu lesen, der mit dem Gasdruck (p_{gin}, p_{gout}) in dem Einlasskanal (4a) oder dem Auslasskanal (4b) oder mit der Gasdruckdifferenz (Δp_{g}) zwischen diesen korrespondiert;
- eine Variationseinrichtung ((23) zum Variieren wenigstens des Gasdruckes im Einlasskanal (4a) und/oder im Auslasskanal (4b), wobei die Variationseinrichtung (23) mit der ersten und der zweiten Sensoreinrichtung (21, 22) operativ verbunden sind und so programmiert sind, dass die Differenz zwischen dem charakteristischem Gaswert (Vg), der **durch** die zweite Sensoreinrichtung (22) gelesen wird, und dem charakteristischem Blutwert (V_{b}), der **durch** die erste Sensoreinrichtung (21) gelesen wird, im Wesentlichen gleich wenigstens einem vordefinierten Wert ist.

2. Gerät (20) nach Anspruch 1, **gekennzeichnet durch** die Tatsache, dass die erste Sensoreinrichtung (21) wenigstens in Verbindung mit dem Blutauslassanschluss (3b) positioniert werden kann, und **durch** die Tatsache, dass die zweite Sensoreinrichtung (22) wenigstens in Verbindung mit dem Gaseinlasskanal (4a) positioniert werden kann, wobei der charakteristische Gaswert (Vg) und der charakteristische Blutwert (V_{b}) mit dem Gaseinlassdruck (p_{gin}) in dem Einlasskanal (4a) und mit dem Blutauslassdruck (p_{bout}) in dem Auslassanschluss (3b) korrespondieren.

3. Gerät (20) nach Anspruch 1 oder 2, **gekennzeichnet durch** die Tatsache, dass die erste Sensoreinrichtung (21) in Verbindung mit dem Bluteinlass- und Auslassanschluss (3a, 3b) positioniert werden kann, um die Blutdruckdifferenz (Δp_{b}) zwischen diesen zu lesen, dass die zweite Sensoreinrichtung (22) in Verbindung mit dem Gaseinlass- und Auslasskanal (4a, 4b) positioniert werden kann, um die Gasdruckdifferenz (Δp_{g}) zwischen diesen zu lesen, wobei der charakteristische Gaswert (Vg) und der charakteristische Blutwert (V_{b}) mit der Gasdruckdifferenz (Δp_{g}) bzw. mit der Blutdruckdifferenz (Δp_{b}) korrespondieren.

4. Gerät (20) nach ein oder mehreren der vorstehenden Ansprüche, **gekennzeichnet durch** die Tatsache, dass der vordefinierte Wert im Wesentlichen größer oder gleich 80mmHg ist.

5. Gerät (20) nach ein oder mehreren der vorhergehenden Ansprüche, **gekennzeichnet durch** die Tatsache, dass dieses eine Befehls- und Steuereinrichtung (25) für die Variationseinrichtung (23) umfasst.

6. Gerät (20) nach Anspruch 5, **gekennzeichnet durch** die Tatsache, dass die Befehls- und Steuereinrichtung (25) aufweist wenigstens einen ersten und zweiten Einlass, der operativ mit der ersten bzw. zweiten Sensoreinrichtung (21, 22) verbunden ist, und wenigstens einen Auslass, der operativ mit der Variationseinrichtung (23) verbunden ist, wobei die Befehls- und Steuereinrichtung (25) die Differenz zwischen dem charakteristischem Gaswert (Vg) und dem charakteristischem Blutwert (V_{b}), der **durch** die erste bzw. **durch** die zweite Sensoreinrichtung (21, 22) gelesen wird, berechnen soll und ein Anzeigesignal für die Differenz an die Variationseinrichtung (23) senden soll.

7. Gerät (20) nach ein oder mehreren der vorhergehenden Ansprüche, **gekennzeichnet durch** die Tatsache, dass die zweite Sensoreinrichtung (22) mit der Befehls- und Steuereinrichtung (25) rückgekoppelt ist.

8. Gerät (20) nach ein oder mehreren der vorhergehenden Ansprüche, **gekennzeichnet durch** die Tatsache, dass die Variationseinrichtung (23) ein Regulationsmittel (24) zum Regulieren des Strömungsquerschnitts wenigstens des Einlasskanals (4a) und/oder des Auslasskanals (4b) umfasst.

9. Gerät (20) nach Anspruch 8, **gekennzeichnet durch** die Tatsache, dass die zweite Sensoreinrichtung (22) in Verbindung mit dem Gaseinlass- und/oder Auslasskanal (4b, 4b) stromabwärts bzw. stromaufwärts der Regulationseinrichtung (24) in Bezug zu der Richtung des Arbeitsgasflusses positioniert werden kann.

10. Gerät (20) nach ein oder mehreren der vorhergehenden Ansprüche, **gekennzeichnet durch** die Tatsache, dass die Variationseinrichtung (23) ein Regulationsmittel zum Regulieren des Gasflusses **durch** den Einlass- und Auslasskanal (4a, 4b) umfasst.

11. Gerät (20) nach ein oder mehreren der vorhergehenden Ansprüche, **gekennzeichnet durch** die Tatsache, dass die Variationseinrichtung (23) wenigstens ein aktives Element umfasst, welches auf einer Förderpumpe zum Zuführen von Blut zur Sauerstoffanreicherungseinrichtung (2) positioniert werden kann und zum Modifizieren des Blutflusses **durch** den Einlass- und Auslassanschluss (3a, 3b) geeignet ist.

12. Gerät (20) nach ein oder mehreren der vorhergehenden Ansprüche, **gekennzeichnet durch** die Tatsache, dass dieses eine Heizeinrichtung zum Erwärmen des in die Sauerstoffanreicherungseinrichtung (2) eintretenden Gases umfasst.

13. Vorrichtung mit:
- einer Einrichtung (2) zur Blut- Sauerstoffanreicherung mit wenigstens einem Einlassanschluss (3a) und einem Auslassanschluss (3b) für das Blut, wenigstens einem Einlasskanal (4a) und einem Auslasskanal (4b) für ein Arbeitsgas, welches Sauerstoff dem Blut zuführen soll und/oder CO₂ von denselben abführen soll, wobei zwischen dem Einlasskanal (4a) und dem Auslasskanal (4b) eine Fließeinrichtung (5) für das Gas angeordnet ist;
- wenigstens einer Förderpumpe, die geeignet ist zum Bewegen des Blutes durch die Sauerstoffanreicheningseinrichtung (2);
wobei die Vorrichtung auch ein Gerät (20) nach ein oder mehreren der Ansprüche 1 bis 12 umfasst, wobei das Gerät (20) mit der Sauerstoffanreicheningseinrichtung (2) stromabwärts mit der Förderpumpe verbunden ist.

## Revendications

1. Equipement (20) apte à être connecté à un dispositif (2) pour l'oxygénation extracorporelle du sang, du type comprenant au moins une liaison d'entrée (3a) et une liaison de sortie (3b) pour le sang, au moins un canal d'entrée (4a) et un canal de sortie (4b) pour un gaz de travail destiné à fournir de l'oxygène au sang et/ou à éliminer le CO₂ de celui-ci, **caractérisé par le fait qu'**il comprend :
- des premiers moyens de capteur (21) qui peuvent être positionnés le long d'au moins l'une des liaisons d'entrée et de sortie (3a, 3b) de sang pour lire au moins une valeur sanguine caractéristique (V_{b}) correspondant à la pression sanguine (p_{bin}, p_{bout}) le long de l'une parmi ladite liaison d'entrée (3a) et ladite liaison de sortie (3b) ou à l'intervalle de pression sanguine (Δp_{b}) entre elles ;
- des deuxièmes moyens de capteur (22) qui peuvent être positionnés le long d'au moins l'un des canaux d'entrée et de sortie (4a, 4b) de gaz pour lire au moins une valeur de gaz caractéristique (Vg) correspondant à la pression de gaz (p_{gin}, p_{gout}) le long de l'un parmi ledit canal d'entrée (4a) et ledit canal de sortie (4b) ou à l'intervalle de pression de gaz (Δp_{g}) entre eux ;
- des moyens de variation (23) pour faire varier au moins la pression de gaz le long du canal d'entrée (4a) et/ou le long du canal de sortie (4b), lesdits moyens de variation (23) étant reliés de manière opérationnelle auxdits premiers et deuxièmes moyens de capteur (21, 22) et étant programmés pour rendre la différence entre ladite valeur de gaz caractéristique (Vg), lue par lesdits deuxièmes moyens de capteur (22), et ladite valeur sanguine caractéristique (V_{b}), lue par lesdits premiers moyens de capteur (21), sensiblement égale à au moins une valeur prédéfinie.

2. Equipement (20) selon la revendication 1, **caractérisé par le fait que** lesdits premiers moyens de capteur (21) peuvent être positionnés au moins le long de ladite liaison de sortie sanguine (3b) et **par le fait que** lesdits deuxièmes moyens de capteur (22) peuvent être positionnés au moins le long dudit canal d'entrée de gaz (4a), lesdites valeur de gaz caractéristique (Vg) et valeur sanguine caractéristique (V_{b}) correspondant respectivement à la pression d'entrée de gaz (p_{gin}) le long dudit canal d'entrée (4a) et à la pression de sortie sanguine (p_{bout}) le long de ladite liaison de sortie (3b).

3. Equipement (20) selon la revendication 1 ou 2, **caractérisé par le fait que** lesdits premiers moyens de capteur (21) peuvent être positionnés le long desdites liaisons d'entrée et de sortie sanguine (3a, 3b) pour lire l'intervalle de pression sanguine (Δp_{b}) entre elles, et que lesdits deuxièmes moyens de capteur (22) peuvent être positionnés le long desdits canaux d'entrée et de sortie de gaz (4a, 4b) pour lire l'intervalle de pression de gaz (Δp_{g}) entre eux, lesdites valeur de gaz caractéristique (Vg) et valeur sanguine caractéristique (V_{b}) correspondant respectivement audit intervalle de pression de gaz (Δp_{g}) et audit intervalle de pression sanguine (Δp_{b}).

4. Equipement (20) selon une ou plusieurs des revendications précédentes, **caractérisé par le fait que** ladite valeur prédéfinie est sensiblement supérieure ou égale à 80 mm Hg.

5. Equipement (20) selon une ou plusieurs des revendications précédentes, **caractérisé par le fait qu'**il comprend des moyens de commande et de contrôle (25) desdits moyens de variation (23).

6. Equipement (20) selon la revendication 5, **caractérisé par le fait que** lesdits moyens de commande et de contrôle (25) comportent au moins une première entrée et une deuxième entrée reliées de manière opérationnelle respectivement auxdits premiers et deuxièmes moyens de capteur (21, 22), et au moins une sortie reliée de manière opérationnelle auxdits moyens de variation (23), lesdits moyens de commande et de contrôle (25) étant destinés à calculer la différence entre ladite valeur de gaz caractéristique (Vg) et ladite valeur sanguine caractéristique (V_{b}) lues respectivement par lesdits premiers et deuxièmes moyens de capteur (21, 22), et à envoyer un signal d'indication de ladite différence auxdits moyens de variation (23).

7. Equipement (20) selon une ou plusieurs des revendications précédentes, **caractérisé par le fait que** lesdits deuxièmes moyens de capteur (22) sont reliés par voie de rétroaction auxdits moyens de commande et de contrôle (25).

8. Equipement (20) selon une ou plusieurs des revendications précédentes, **caractérisé par le fait que** lesdits moyens de variation (23) comprennent des moyens de régulation (24) pour réguler la section d'écoulement d'au moins l'un dudit canal d'entrée (4a) et dudit canal de sortie (4b).

9. Equipement (20) selon la revendication 8, **caractérisé par le fait que** lesdits deuxièmes moyens de capteur (22) peuvent être positionnés le long desdits canaux d'entrée et/ou de sortie de gaz (4a, 4b), respectivement en aval et en amont des moyens de régulation (24) par rapport à la direction d'écoulement du gaz de travail.

10. Equipement (20) selon une ou plusieurs des revendications précédentes, **caractérisé par le fait que** lesdits moyens de variation (23) comprennent des moyens de régulation pour réguler l'écoulement de gaz à travers lesdits canaux d'entrée et de sortie (4a, 4b).

11. Equipement (20) selon une ou plusieurs des revendications précédentes, **caractérisé par le fait que** lesdits moyens de variation (23) comprennent au moins un élément actif qui peut être positionné sur une pompe d'alimentation fournissant du sang au dispositif d'oxygénation (2) et qui est apte à modifier le flux sanguin à travers lesdites liaisons d'entrée et de sortie (3a, 3b).

12. Equipement (20) selon une ou plusieurs des revendications précédentes, **caractérisé par le fait qu'**il comprend des moyens de chauffage pour chauffer le gaz entrant dans ledit dispositif d'oxygénation (2).

13. Appareil comprenant :
- un dispositif (2) pour l'oxygénation du sang comportant au moins une liaison d'entrée (3a) et une liaison de sortie (3b) pour le sang, au moins un canal d'entrée (4a) et un canal de sortie (4b) d'un gaz de travail destiné à fournir de l'oxygène au sang et/ou à éliminer le CO₂ de celui-ci, des moyens d'écoulement (5) pour le gaz étant disposés entre ledit canal d'entrée (4a) et ledit canal de sortie (4b) ;
- au moins une pompe d'alimentation apte à déplacer le sang à travers ledit dispositif d'oxygénation (2) ;
ledit appareil comprenant également un équipement (20) selon une ou plusieurs des revendications 1 à 12, ledit équipement (20) étant relié audit dispositif d'oxygénation (2) en aval de ladite pompe d'alimentation.
